# EUROPEAN PATENT SPECIFICATION

(11) **EP 2 546 259 B1**
(45) Date of publication and mention of the grant of the patent: **14.10.2015**
(21) Application number: 11752900.8
(22) Date of filing: 10.03.2011
(51) Int. Cl.: C07H 15/04

(54) **METHOD FOR OBTAINING BIODEGRADABLE SURFACTANTS FROM CELLULOSE IN A SINGLE REACTOR**
VERFAHREN ZUR GEWINNUNG BIOLOGISCH ABBAUBARER TENSIDE AUS ZELLULOSE IN EINEM EINZELREAKTOR
PROCÉDÉ D'OBTENTION DE TENSIOACTIFS BIODÉGRADABLES À PARTIR DE CELLULOSE DANS UN SEUL RÉACTEUR

(30) Priority: 12.03.2010 ES 201030368
(43) Date of publication of application: 16.01.2013
(73) Proprietor: Consejo Superior De Investigaciones Científicas (CSIC), 28006 Madrid (ES); Universidad Politecnica de Valencia, 46022 Valencia (ES)
(72) Inventor: CORMA CANOS, Avelino, E-46022 Valencia (ES); VILLANDIER, Nicolás David, E-46022 Valencia (ES)
(74) Representative: Ungria López, Javier
(86) International application number: PCT/ES2011/070162
(87) International publication number: WO 2011/110721

(56) References cited:
- ES-T3- 2 154 639
- US-A- 3 450 690
- US-A- 3 839 318
- US-A- 4 987 225
- ROBERTO RINALDI ET AL: "Depolymerization of Cellulose Using Solid Catalysts in Ionic Liquids", ANGEWANDTE CHEMIE. INTERNATIONAL EDITION, WILEY VCH VERLAG, WEINHEIM, vol. 47, no. 42, 12 September 2008 (2008-09-12), pages 8047-8050, XP009106605, ISSN: 1433-7851, DOI: 10.1002/ANIE.200802879
- NICOLAS VILLANDIER ET AL: "One pot catalytic conversion of cellulose into biodegradable surfactants", CHEMICAL COMMUNICATIONS, vol. 46, no. 24, 18 May 2010 (2010-05-18), page 4408, XP055087999, ISSN: 1359-7345, DOI: 10.1039/c0cc00031k
- NICOLAS VILLANDIER ET AL: "Transformation of Cellulose into Biodegradable Alkyl Glycosides by Following Two Different Chemical Routes", CHEMSUSCHEM, vol. 4, no. 4, 9 March 2011 (2011-03-09), pages 508-513, XP055088001, ISSN: 1864-5631, DOI: 10.1002/cssc.201000371
- DENG, W. ET AL.: 'Acid-catalysed direct transformation of cellulose into methyl glucosides in methanol at moderate temperatures' CHEMICAL COMMUNICATIONS vol. 46, 06 February 2010, pages 2668 - 2670, XP055088062

## Description

### FIELD OF THE INVENTION

The present invention belongs to the field of "one pot" methods for catalytic conversion of cellulose into alkyl-α,β-glycosides.

### STATE OF THE ART PRIOR TO THE INVENTION

The current growing demand for reducing carbon dioxide emissions into the atmosphere because of its effect on global warming, has generated a rapid development of alternative technologies that use renewable raw materials among which is cellulose. The fact that cellulose is abundant and is a renewable source, has led to a boom in studies related to the transformation of the same.

On the other hand, the long-chain alkyl glycosides are non-ionic compounds that have excellent properties as surfactants, as well as having a low toxicity and being biodegradable. These carbohydrates derivatives can be used in cosmetics and detergents, as emulsifiers in the food industry and as dispersing agents in pharmaceutics.

There are two main methods for obtaining this type of compounds, the Fischer glycosidation process and the Koenig-Knorr method. The Fisher glycosidation process is simpler and less expensive than the Koenig-Knorr method and involves an acetylation of a carbohydrate (usually glucose) using an acid catalyst and in the presence of an alcohol. Various acid catalysts, both homogeneous and heterogeneous, are described in the literature that have been used, such as, e.g. ionic exchange resins, amorphous silica-alumina, zeolite and mesoporous materials of the MCM-41 type, mineral catalysts and organic acids among others.

Acid hydrolysis of the cellulose is an important source for obtaining glucose. Cellulose, which as mentioned above is an increasingly important source for obtaining biofuels and chemical compounds, is a crystalline polymer of D-glucopyranose units joined together through β-1,4-glycosidic bonds. The interaction between different chains is ensured through hydrogen bonds and Van der Valls interactions, which provides cellulose with a high stability, making it difficult to carry out the process of hydrolysis of the same.

Most of the processes of hydrolysis to convert the cellulose into glucose are carried out in water. The reaction is carried out in the presence of acid minerals, enzymes or under hydrothermal conditions. Recently, processes using heterogeneous catalysts have been developed. S. Sugama et al, J. Amer. Chem. Soc. 2008 describes the use of carbon materials with SO₃H groups. A. Takagaky et al, Chem. Comm. 2008, use laminated metal oxides such as, e.g. HNbMoO₆, but the yield of glucose is too low in both cases. To promote the transformation of cellulose into glucose, Onda et al, Green Chemistry, 2008 and Top. Cat. 2009, use as starting material cellulose that has been pre-treated to reduce the crystallinity of the same. R.P. Swatloski, J. Amer. Chem. Soc. 2002, have described that it is possible to dissolve cellulose in ionic liquids, and also in the presence of mineral catalysts or acid solid catalysts cellulose can also be depolymerized.

W. Deng et al, Chem. Comm. 2010 have described the transformation of cellulose into methyl-α,β-glycosides in methanol medium with a yield of 50%-60% in the presence of several acid catalysts, at 468 K and 30 bars. But this process requires high pressure and temperature, and affords alkyl-glucosides low yields when it reacts with long-chain alcohols needed to obtain products with surfactant properties such as those obtained by the method of the present invention.

The present invention describes a method able to convert cellulose into alkyl-α,β-glycosides surfactants in mild conditions and in a reaction in a single reactor using an appropriate catalyst and suitable reaction conditions that allow to couple the hydrolysis of the starting cellulose with the Fisher glycosidation of the glucose formed in the first step with alcohols with chains with more than 4 carbons. Processes in a single reactor (also known as *one pot* reactions) are a strategy intended to intensify the processes to improve the efficiency of reactions that occur in series. These reactions are being widely studied because of their numerous advantages such as the elimination of the processes of separation and purification of intermediates with the subsequent increase in production and reduction of investment and waste formation.

### DESCRIPTION OF THE INVENTION

The present invention relates to a method for obtaining surfactants from cellulose and hemicellulose that is carried out in a reaction in a single reactor, *one pot,* and that comprises at least the following steps:
a) a first step of hydrolysis where the cellulose is mixed with at least one ionic liquid, water and catalyst;
b) a second step of glycosidation wherein at least one alcohol is added when the hydrolysis level of the cellulose is comprised between 10 and 80%.

When cellulose is referred to in the description of the present invention, it refers both to cellulose and mixtures of hemicellulose and cellulose.

According to this method, it is possible to transform cellulose directly into surfactants, e.g. alkyl-α,β-glycosides, preferably alkyl-α,β-glucosides and alkyl-α,β-xylosides in a *one pot* reaction, and for this, the appropriate catalyst and the appropriate reaction conditions that allow to couple the two reactions that have to be carried out in *one pot* have been found, the hydrolysis of the starting cellulose in ionic medium and the Fisher glycosidation of the glucose formed in the first step with alcohols.

Preferably, the ionic liquid that is used in the first step can be preferably selected from ionic liquids that contain the imidazolium group as cation, and more preferably it is BMIMCI.

In the method described according to the present invention, the amount of water present in the medium is important since, although it favours the first stage of hydrolysis of the cellulose and minimizes the formation of HMF (unwanted product), at the same time it has a negative effect on the second stage of glycosidation of glucose, so it is necessary to find an amount which is most conducive to the hydrolysis of the cellulose trying to cause the least possible negative effect on the Fisher glycosidation. As explained above, according to a particular embodiment of the present invention the cellulose/water ratio is between 20 and 0.2 by weight, more preferably between 10 and 0.5 by weight.

Both for the hydrolysis of cellulose and the Fisher glycosidation of the method that is carried out according to the present invention is necessary that the catalyst is an acid catalyst. This catalyst can be preferably selected from heteropoly acids and catalysts comprising sulfonic groups. The heteropoly acids are preferred heteropoly acids containing PO₄ or SiO₄ tetrahedra. In addition, these heteropoly acids contain preferably Mo o W. According to a particular embodiment, the heteropoly acid is H₃PW₁₂O₄₀.

In the case where the catalyst comprises sulfonic groups, they must be accessible so as to increase the effectiveness of the catalyst. According to a particular embodiment, the catalyst used involves the use of resin with sulfonic groups, being an example of them Amberlyst 15Dry. Amberlyst 15Dry resin is a commercial catalyst from Rohm and Haas company.

Once carried out the first step, in which the initial cellulose and the ionic liquid have been mixed together with the catalyst and a certain amount of water, the hydrolysis reaction starts that can be carried out at a preferred temperature between 60 and 140° C and at a preferred pressure between 1 and 5 bars and more preferably is carried out at atmospheric pressure for a period of time sufficient to hydrolyze, preferably between 10% of the cellulose, but no more than 80%. At this time, in the second step, an alcohol is added and the pressure of the system is reduced to a preferred range between 5 and 700 mbar and more preferably between 20 and 600 mbar, maintaining the preferred temperature between 60 and 140° C. In the second stage of the reaction, the hydrolysis of the cellulose can continue taking place at the same time that the second step glycosidation occurs. The reaction time varies depending on the reaction conditions and the amount of catalyst used. Under preferred reaction conditions, the ratio of cellulose or mixtures of cellulose and hemicellulose with respect to ionic liquid can vary between 0.4 and 0.02 by weight, the ratio of cellulose plus ionic liquid to catalyst being preferably between 80 and 5 by weight and preferably between 60 and 10 by weight.

It should be noted that the single combination of results reported so far on hydrolysis of cellulose and glycosidation of glucose with alcohols is not enough for obtaining the results of the process that is described in the present invention. If one carries out the complete hydrolysis of the cellulose and then it is reacted with the alcohol, either at atmospheric pressure or under vacuum, the glycosidation product selectivity is low. If on the other hand the cellulose and the alcohol are mixed from the beginning, the final yield is low. In our case, we have found that surprisingly the results obtained are better when the alcohol is introduced when only a part of cellulose has been hydrolyzed, preferably between 10% and 80%. Moreover, it has been observed that the method is efficient if at that moment is carried out a variation of the working pressure in the above ranges.

The alcohol introduced in step 2, is preferably an alcohol with 4 or more carbons, such as for example butanol and hexanol and more preferably is an alcohol with 8 or more carbons, such as for example, octanol, decanol dodecanol and tetradecanol, preferably octanol. Preferably, these alcohols can be linear alcohols.

The products obtained according to the method of the present invention may be alkyl-α,β-glycosides, preferably alkyl-α,β-glycosides and alkyl-α,β-xylosides that can be used as surfactants due to their properties.

Throughout the description and the claims the word "comprises" and its variants are not intended to exclude other technical features, additives, components or steps. For the skilled in the art, other objects, advantages and features of the invention will derive in part from the description and in part from the practice of the invention. The following examples are provided by way of illustration, and are not intended to be limiting of the present invention.

### EMBODIMENTS OF THE INVENTION

### EXAMPLES

### Example 1:

0.3 g of α-cellulose and 6 g of BMIMCI are introduced in a container and heated at 100° C, at atmospheric pressure until a clear solution is formed (about 30 minutes). 315 mg of water and 160 mg of Amberlyst 15Dry catalyst are added to this mixture. Stir vigorously. After 1.5 hours 7 ml of octanol are added and stirred vigorously at 90° C. The reaction is carried out at a pressure of 40 mbar for 24 hrs.

The total yield to surfactants is of 81.7% by weight, 70% corresponding to alkyl-α,β-glucoside and 11.7% to alkyl-α,β-xyloside.

### Example 2:

0.3 g of α-cellulose and 6 g of BMIMCI are introduced in a container and heated at 100° C, at atmospheric pressure until a clear solution is formed (about 30 minutes). 315 mg of water and 160 mg of Amberlyst 15Dry catalyst are added to this mixture. Stir vigorously. After 1.5 hours 5.5 ml of hexanol are added and stirred vigorously at 90° C. The reaction is carried out at a pressure of 40 mbar for 24 hrs.

The total yield to surfactants is of 72.4% by weight, 60.1 % corresponding to alkyl-α,β-glucoside and 12.3% to alkyl-α,β-xyloside.

### Example 3:

0.3 g of cellulose fibre and 6 g of BMIMCI are introduced in a container and heated at 100° C, at atmospheric pressure until a clear solution is formed (about 30 minutes). 760 mg of water and 350 mg of Amberlyst 15Dry catalyst are added to this mixture. Stir vigorously. After 40 minutes 8 ml of octanol are added and stirred vigorously at 90° C. The reaction is carried out at a pressure of 40 mbar for 24 hrs.

The total yield to surfactants is of 71.5% by weight, 71.5% corresponding to alkyl-α,β-glucoside.

### Example 4:

0.3 g of α-cellulose and 6 g of BMIMCI are introduced in a container and heated at 100° C, at atmospheric pressure until a clear solution is formed (about 30 minutes). 760 mg of water and 710 mg of H₃PW₁₂O₄₀ catalyst are added to this mixture. Stir vigorously. After 60 minutes 8 ml of octanol are added and stirred vigorously at 90° C. The reaction is carried out at a pressure of 40 mbar for 24 hrs.

The total yield to surfactants is of 74.9% by weight, 64.0% corresponding to alkyl-α,β-glucoside and 10.9% to alkyl-α,β-xyloside.

### Example 5:

0.3 g of α-cellulose and 6 g of BMIMCI are introduced in a container and heated at 100° C, at atmospheric pressure until a clear solution is formed (about 30 minutes). 315 mg of water and 160 mg of Amberlyst 15Dry catalyst are added to this mixture. Stir vigorously. After 5 hours 8 ml of octanol are added and stirred vigorously at 90° C. The reaction is carried out at a pressure of 40 mbar for 24 hrs.

The total yield to surfactants is of 48.3% by weight, 43.8% corresponding to alkyl-α,β-glucoside and 4.5% to alkyl-α,β-xyloside.

## Claims

1. Method for obtaining surfactants in a single reactor, *one pot,* from cellulose and hemicellulose comprising at least:
a) a first step of hydrolysis where the cellulose is mixed with at least one ionic liquid, and with catalyst;
b) a second step of glycosidation wherein at least one alcohol is added when the hydrolysis level of the cellulose is comprised between 10 and 80%.

2. Method for obtaining surfactants in a single reactor, *one pot,* from cellulose and hemicellulose according to claim 1, wherein the first step is carried out at a pressure between 1 and 5 bars.

3. Method for obtaining surfactants in a single reactor, *one pot,* from cellulose and hemicellulose according to claim 2, wherein the first step is carried out at atmospheric pressure.

4. Method for obtaining surfactants in a single reactor, *one pot,* from cellulose and hemicellulose according to claim 1, wherein the second step is carried out at a pressure between 5 and 700 mbar.

5. Method for obtaining surfactants in a single reactor, *one pot,* from cellulose and hemicellulose according to claim 4, wherein the second step is carried out at a pressure between 20 and 600 mbar.

6. Method for obtaining surfactants in a single reactor, *one pot,* from cellulose and hemicellulose according to any of the previous claims, wherein it is carried out at a temperature between 60 and 140° C.

7. Method for obtaining surfactants in a single reactor, *one pot,* from cellulose and hemicellulose according to claim 1, wherein the ionic liquid of the first step is selected from ionic liquids containing the imidazolium group as a cation.

8. Method for obtaining surfactants in a single reactor, *one pot,* from cellulose and hemicellulose according to claim 7, wherein the ionic liquid is BMIMCI.

9. Method for obtaining surfactants in a single reactor, *one pot,* from cellulose and hemicellulose according to claim 1, wherein the cellulose/water ratio is between 20 and 0.2 by weight.

10. Method for obtaining surfactants in a single reactor, *one pot,* from cellulose and hemicellulose according to claim 9, wherein the cellulose/water ratio is between 10 and 0.5 by weight.

11. Method for obtaining surfactants in a single reactor, *one pot,* from cellulose and hemicellulose according to claim 1, wherein the ratio between cellulose and ionic liquid is between 0.4 and 0.02 by weight.

12. Method for obtaining surfactants in a single reactor, *one pot,* from cellulose and hemicellulose according to claim 1, wherein the ratio of cellulose plus ionic liquid to catalyst is between 80 and 5 by weight.

13. Method for obtaining surfactants in a single reactor, *one pot,* from cellulose and hemicellulose according to claim 12, wherein the ratio of cellulose plus ionic liquid to catalyst is between 60 and 10 by weight.

14. Method for obtaining surfactants in a single reactor, *one pot,* from cellulose and hemicellulose according to claim 1, **characterized in that** the catalyst is an acid catalyst.

15. Method for obtaining surfactants in a single reactor, *one pot,* from cellulose and hemicellulose according to claim 14, wherein the catalyst comprises sulfonic groups.

16. Method for obtaining surfactants in a single reactor, *one pot,* from cellulose and hemicellulose according to claim 15, wherein said catalyst is a resin.

17. Method for obtaining surfactants in a single reactor, *one pot,* from cellulose and hemicellulose according to claim 16, wherein the resin is Amberlyst 15Dry.

18. Method for obtaining surfactants in a single reactor, *one pot,* from cellulose and hemicellulose according to claim 14, wherein the catalyst is a heteropoly acid.

19. Method for obtaining surfactants in a single reactor, *one pot,* from cellulose and hemicellulose according to claim 18, wherein the heteropoly acid contains PO₄ or SiO₄ tetrahedra.

20. Method for obtaining surfactants in a single reactor, *one pot,* from cellulose and hemicellulose according to one of claims 18 and 19, wherein the heteropoly acid contains Mo or W.

21. Method for obtaining surfactants in a single reactor, *one pot,* from cellulose and hemicellulose according to claim 1, wherein the alcohol is an alcohol with 4 or more carbons.

22. Method for obtaining surfactants in a single reactor, *one pot,* from cellulose and hemicellulose according to claim 21, wherein the alcohol is an alcohol with 8 or more carbons.

23. Method for obtaining surfactants in a single reactor, *one pot,* from cellulose and hemicellulose according to claim 22, wherein the alcohol is octanol.

24. Method for obtaining surfactants in a single reactor, *one pot,* from cellulose and hemicellulose according to one of claims 21 to 23, wherein the alcohol is linear.

## Patentansprüche

1. Verfahren zum Erhalten von oberflächenaktiven Stoffen in einem einzelnen (Eintopf-) Reaktor aus Cellulose und Hemicellulose, umfassend wenigstens:
a) einen ersten Schritt einer Hydrolyse, wobei die Cellulose mit wenigstens einer ionischen Flüssigkeit und mit einem Katalysator vermischt wird;
b) einen zweiten Schritt einer Glycosidierung, wobei wenigstens ein Alkohol zugegeben wird, wenn das Hydrolyseniveau der Cellulose zwischen 10 und 80 % liegt.

2. Verfahren zum Erhalten von oberflächenaktiven Stoffen in einem einzelnen (Eintopf-) Reaktor aus Cellulose und Hemicellulose nach Anspruch 1, wobei der erste Schritt bei einem Druck zwischen 1 und 5 bar durchgeführt wird.

3. Verfahren zum Erhalten von oberflächenaktiven Stoffen in einem einzelnen (Eintopf-) Reaktor aus Cellulose und Hemicellulose nach Anspruch 2, wobei der erste Schritt bei Atmosphärendruck durchgeführt wird.

4. Verfahren zum Erhalten von oberflächenaktiven Stoffen in einem einzelnen (Eintopf-) Reaktor aus Cellulose und Hemicellulose nach Anspruch 1, wobei der zweite Schritt bei einem Druck zwischen 5 und 700 mbar durchgeführt wird.

5. Verfahren zum Erhalten von oberflächenaktiven Stoffen in einem einzelnen (Eintopf-) Reaktor aus Cellulose und Hemicellulose nach Anspruch 4, wobei der zweite Schritt bei einem Druck zwischen 20 und 600 mbar durchgeführt wird.

6. Verfahren zum Erhalten von oberflächenaktiven Stoffen in einem einzelnen (Eintopf-) Reaktor aus Cellulose und Hemicellulose nach einem der vorangehenden Ansprüche, wobei es bei einer Temperatur zwischen 60 und 140 °C durchgeführt wird.

7. Verfahren zum Erhalten von oberflächenaktiven Stoffen in einem einzelnen (Eintopf-) Reaktor aus Cellulose und Hemicellulose nach Anspruch 1, wobei die ionische Flüssigkeit des ersten Schritts aus ionischen Flüssigkeiten ausgewählt ist, die die Imidazoliumgruppe als Kation enthalten.

8. Verfahren zum Erhalten von oberflächenaktiven Stoffen in einem einzelnen (Eintopf-) Reaktor aus Cellulose und Hemicellulose nach Anspruch 7, wobei die ionische Flüssigkeit BMIMCI ist.

9. Verfahren zum Erhalten von oberflächenaktiven Stoffen in einem einzelnen (Eintopf-) Reaktor aus Cellulose und Hemicellulose nach Anspruch 1, wobei das Cellulose/Wasser-Verhältnis zwischen 20 und 0,2, bezogen auf das Gewicht, liegt.

10. Verfahren zum Erhalten von oberflächenaktiven Stoffen in einem einzelnen (Eintopf-) Reaktor aus Cellulose und Hemicellulose nach Anspruch 9, wobei das Cellulose/Wasser-Verhältnis zwischen 10 und 0,5, bezogen auf das Gewicht, liegt.

11. Verfahren zum Erhalten von oberflächenaktiven Stoffen in einem einzelnen (Eintopf-) Reaktor aus Cellulose und Hemicellulose nach Anspruch 1, wobei das Verhältnis zwischen Cellulose und ionischer Flüssigkeit zwischen 0,4 und 0,02, bezogen auf das Gewicht, liegt.

12. Verfahren zum Erhalten von oberflächenaktiven Stoffen in einem einzelnen (Eintopf-) Reaktor aus Cellulose und Hemicellulose nach Anspruch 1, wobei das Verhältnis von Cellulose plus ionischer Flüssigkeit zu Katalysator zwischen 80 und 5, bezogen auf das Gewicht, liegt.

13. Verfahren zum Erhalten von oberflächenaktiven Stoffen in einem einzelnen (Eintopf-) Reaktor aus Cellulose und Hemicellulose nach Anspruch 12, wobei das Verhältnis von Cellulose plus ionischer Flüssigkeit zu Katalysator zwischen 60 und 10, bezogen auf das Gewicht, liegt.

14. Verfahren zum Erhalten von oberflächenaktiven Stoffen in einem einzelnen (Eintopf-) Reaktor aus Cellulose und Hemicellulose nach Anspruch 1, **dadurch gekennzeichnet, dass** der Katalysator ein Säurekatalysator ist.

15. Verfahren zum Erhalten von oberflächenaktiven Stoffen in einem einzelnen (Eintopf-) Reaktor aus Cellulose und Hemicellulose nach Anspruch 14, wobei der Katalysator Sulfonsäuregruppen umfasst.

16. Verfahren zum Erhalten von oberflächenaktiven Stoffen in einem einzelnen (Eintopf-) Reaktor aus Cellulose und Hemicellulose nach Anspruch 15, wobei der Katalysator ein Harz ist.

17. Verfahren zum Erhalten von oberflächenaktiven Stoffen in einem einzelnen (Eintopf-) Reaktor aus Cellulose und Hemicellulose nach Anspruch 16, wobei das Harz Amberlyst 15Dry ist.

18. Verfahren zum Erhalten von oberflächenaktiven Stoffen in einem einzelnen (Eintopf-) Reaktor aus Cellulose und Hemicellulose nach Anspruch 14, wobei der Katalysator eine Heteropolysäure ist.

19. Verfahren zum Erhalten von oberflächenaktiven Stoffen in einem einzelnen (Eintopf-) Reaktor aus Cellulose und Hemicellulose nach Anspruch 18, wobei die Heteropolysäure PO₄ oder SiO₄ Tetraeder enthält.

20. Verfahren zum Erhalten von oberflächenaktiven Stoffen in einem einzelnen (Eintopf-) Reaktor aus Cellulose und Hemicellulose nach einem der Ansprüche 18 und 19, wobei die Heteropolysäure Mo oder W enthält.

21. Verfahren zum Erhalten von oberflächenaktiven Stoffen in einem einzelnen (Eintopf-) Reaktor aus Cellulose und Hemicellulose nach Anspruch 1, wobei der Alkohol ein Alkohol mit 4 oder mehr Kohlenstoffatomen ist.

22. Verfahren zum Erhalten von oberflächenaktiven Stoffen in einem einzelnen (Eintopf-) Reaktor aus Cellulose und Hemicellulose nach Anspruch 21, wobei der Alkohol ein Alkohol mit 8 oder mehr Kohlenstoffatomen ist.

23. Verfahren zum Erhalten von oberflächenaktiven Stoffen in einem einzelnen (Eintopf-) Reaktor aus Cellulose und Hemicellulose nach Anspruch 22, wobei der Alkohol Octanol ist.

24. Verfahren zum Erhalten von oberflächenaktiven Stoffen in einem einzelnen (Eintopf-) Reaktor aus Cellulose und Hemicellulose nach einem der Ansprüche 21 bis 23, wobei der Alkohol linear ist.

## Revendications

1. Procédé d'obtention de tensioactifs dans un réacteur unique, monopote, à partir de cellulose et d'hémicellulose comprenant au moins :
a) une première étape d'hydrolyse dans laquelle la cellulose est mélangée avec au moins un liquide ionique, et avec un catalyseur ;
b) une deuxième étape de glycosidation dans laquelle au moins un alcool est ajouté lorsque le taux d'hydrolyse de la cellulose est compris entre 10 et 80 %.

2. Procédé d'obtention de tensioactifs dans un réacteur unique, monopote, à partir de cellulose et d'hémicellulose selon la revendication 1, dans lequel la première étape est conduite à une pression comprise entre 1 et 5 bar.

3. Procédé d'obtention de tensioactifs dans un réacteur unique, monopote, à partir de cellulose et d'hémicellulose selon la revendication 2, dans lequel la première étape est conduite à pression atmosphérique.

4. Procédé d'obtention de tensioactifs dans un réacteur unique, monopote, à partir de cellulose et d'hémicellulose selon la revendication 1, dans lequel la deuxième étape est conduite à une pression comprise entre 5 et 700 mbar.

5. Procédé d'obtention de tensioactifs dans un réacteur unique, monopote, à partir de cellulose et d'hémicellulose selon la revendication 4, dans lequel la deuxième étape est conduite à une pression comprise entre 20 et 600 mbar.

6. Procédé d'obtention de tensioactifs dans un réacteur unique, monopote, à partir de cellulose et d'hémicellulose selon l'une quelconque des revendications précédentes, **caractérisé en ce qu'**il est conduit à une température comprise entre 60 et 140 °C.

7. Procédé d'obtention de tensioactifs dans un réacteur unique, monopote, à partir de cellulose et d'hémicellulose selon la revendication 1, dans lequel le liquide ionique de la première étape est choisi parmi des liquides ioniques contenant le groupe imidazolium en tant que cation.

8. Procédé d'obtention de tensioactifs dans un réacteur unique, monopote, à partir de cellulose et d'hémicellulose selon la revendication 7, dans lequel le liquide ionique est BMIMCI.

9. Procédé d'obtention de tensioactifs dans un réacteur unique, monopote, à partir de cellulose et d'hémicellulose selon la revendication 1, dans lequel le rapport cellulose/eau est compris entre 20 et 0,2 en poids.

10. Procédé d'obtention de tensioactifs dans un réacteur unique, monopote, à partir de cellulose et d'hémicellulose selon la revendication 9, dans lequel le rapport cellulose/eau est compris entre 10 et 0,5 en poids.

11. Procédé d'obtention de tensioactifs dans un réacteur unique, monopote, à partir de cellulose et d'hémicellulose selon la revendication 1, dans lequel le rapport entre la cellulose et le liquide ionique est compris entre 0,4 et 0,02 en poids.

12. Procédé d'obtention de tensioactifs dans un réacteur unique, monopote, à partir de cellulose et d'hémicellulose selon la revendication 1, dans lequel le rapport de la cellulose plus le liquide ionique au catalyseur est compris entre 80 et 5 en poids.

13. Procédé d'obtention de tensioactifs dans un réacteur unique, monopote, à partir de cellulose et d'hémicellulose selon la revendication 12, dans lequel le rapport de la cellulose plus le liquide ionique au catalyseur est compris entre 60 et 10 en poids.

14. Procédé d'obtention de tensioactifs dans un réacteur unique, monopote, à partir de cellulose et d'hémicellulose selon la revendication 1, **caractérisé en ce que** le catalyseur est un catalyseur acide.

15. Procédé d'obtention de tensioactifs dans un réacteur unique, monopote, à partir de cellulose et d'hémicellulose selon la revendication 14, dans lequel le catalyseur comprend des groupes sulfoniques.

16. Procédé d'obtention de tensioactifs dans un réacteur unique, monopote, à partir de cellulose et d'hémicellulose selon la revendication 15, dans lequel ledit catalyseur est une résine.

17. Procédé d'obtention de tensioactifs dans un réacteur unique, monopote, à partir de cellulose et d'hémicellulose selon la revendication 16, dans lequel la résine est Amberlyst 15Dry.

18. Procédé d'obtention de tensioactifs dans un réacteur unique, monopote, à partir de cellulose et d'hémicellulose selon la revendication 14, dans lequel le catalyseur est un hétéropolyacide.

19. Procédé d'obtention de tensioactifs dans un réacteur unique, monopote, à partir de cellulose et d'hémicellulose selon la revendication 18, dans lequel l'hétéropolyacide contient un tétraèdre de PO₄ OU SiO₄.

20. Procédé d'obtention de tensioactifs dans un réacteur unique, monopote, à partir de cellulose et d'hémicellulose selon l'une des revendications 18 et 19, dans lequel l'hétéropolyacide contient Mo ou W.

21. Procédé d'obtention de tensioactifs dans un réacteur unique, monopote, à partir de cellulose et d'hémicellulose selon la revendication 1, dans lequel l'alcool est un alcool avec 4 carbones ou plus.

22. Procédé d'obtention de tensioactifs dans un réacteur unique, monopote, à partir de cellulose et d'hémicellulose selon la revendication 21, dans lequel l'alcool est un alcool avec 8 carbones ou plus.

23. Procédé d'obtention de tensioactifs dans un réacteur unique, monopote, à partir de cellulose et d'hémicellulose selon la revendication 22, dans lequel l'alcool est l'octanol.

24. Procédé d'obtention de tensioactifs dans un réacteur unique, monopote, à partir de cellulose et d'hémicellulose selon l'une des revendications 21 à 23, dans lequel l'alcool est linéaire.
